# EUROPEAN PATENT SPECIFICATION

(11) **EP 2 127 603 B1**
(45) Date of publication and mention of the grant of the patent: **17.11.2010**
(21) Application number: 08157317.2
(22) Date of filing: 30.05.2008
(51) Int. Cl.: A61B 10/02

(54) **Device for taking cytologic samples of the cervix**
Gerät zur Entnahme von cytologischen Proben aus dem Uterushalsus
Dispositif de prélévement d'échantillons cytologiques du col de l'utérus

(43) Date of publication of application: 02.12.2009
(73) Proprietor: ACITS 06, S.L., 07340 Alaró, Illes Balears (ES)
(72) Inventor: Rey Lazaro, Máximino, 07340, Alaró, Illes Balears (ES); Gil Bretones, Arturo, 07340, Alaró, Illes Balears (ES)
(74) Representative: Pons Ariño, Angel

(56) References cited:
- WO-A-87/02877
- WO-A-2006/065006
- US-A- 5 129 402
- US-A1- 2005 288 606

## Description

### OBJECT OF THE INVENTION

The main object of this invention is a device designed for patients themselves to autonomously collect cervical smear samples.

### BACKGROUND OF THE INVENTION

Cervical smears are the most effective preventive method to reduce mortality due to cervical cancer. It consists of the extraction of cell samples from the uterine cervix and the subsequent analysis thereof in a laboratory in order to detect the human papillomavirus, or HPV, which is the causing agent in most cancer cases.

However, in the prior art a specialist physician must take a cervical smear from the endocervix with the help of a brush and a spatula. Subsequently, the sample is rubbed against a glass slide in order to view it under a microscope using Papanicolau staining.

One drawback of this preventive method is that it requires patients to visit a specialist in order for the samples to be extracted. Due to shyness or to the time involved in making an appointment with the specialist and in the appointment itself, many women do not attend early cancer detection campaigns.

Also, US patent 5,129,402 describes a specimen collector including an elongated outer hollow tube having a forward end and a rearward end, and an inner elongated rod having a forward and a rearward end portions slidably mounted within the outer tube.

### DESCRIPTION OF THE INVENTION

The device of the invention facilitates the extraction of a sample of uterine cervix cells by the patient herself in a comfortable, quick and reliable manner. Moreover, the sampling device is simple and easy to manufacture. In general, it is a cylindrical device designed to be introduced into the patient's vagina and cause the detachment of uterine cervix cells by rotating the device.

In this description, in order to refer to the various sections of the parts that make up the device of the invention, the length is divided into sections with different characteristics, each of which is called a "portion". On the other hand, the term "end" refers solely to the tip of the part in question. Furthermore, in this document, the terms "proximal" and "distal" have the meaning that they are usually assigned in the field of medicine.

This invention discloses a uterine cervix sampling device to be used by the patient herself, which comprises three parts: a cylindrical thimble, a cannula and a sheath. Each part is disclosed in detail below:

1) A hollow cylindrical thimble the exterior surface whereof comprises sampling means, and the distal end whereof is closed by an anatomical surface.

The thimble is the part of the device where the cervical smear sampling is performed and, therefore, it is made of a soft, non-aggressive, biocompatible material. In preferred embodiments of the invention, the material is biocompatible polyethylene.

The thimble has the shape of a hollow cylinder between 30 and 50 mm in length and between 10 and 20 mm in diameter. The distal end is closed by an anatomical surface suitable to be introduced through the vagina without causing damage or discomfort. Preferably, the anatomical surface is semi-spherical, although other shapes are possible, provided that they are comfortable and prevent damages upon penetrating along the uterine cervix. The opposite end of the thimble, that is, the proximal end, is open.

The interior surface of the thimble preferably comprises a fastening means to a cannula (which is disclosed further below in this document) and, more preferably, comprises a first flange located on the proximal end.

The exterior surface of the thimble comprises sampling means, which, in a preferred embodiment of the invention, consist of a plurality of protuberances which, upon rubbing the thimble against the uterine cervix walls, cause some cells to be detached. The protuberances may have any shape provided that this objective is achieved without causing damage to the uterine cervix walls, although preferably they are semi-spherical.

2) An elongated cannula, formed by a distal portion, a central portion and a proximal portion, the distal portion whereof is coupled to the interior of the thimble through a supplementary fastening means.

The distal portion of the cannula is coupled to the thimble and helps to introduce a sufficient length thereof into the vaginal cavity in order to reach the uterine cervix, where the sample is collected. The cannula has three clearly distinct parts:

The shape of the distal portion of the cannula complements that of the interior of the thimble and, in a preferred embodiment of the invention, additionally comprises a supplementary fastening means to the thimble. The supplementary fastening means to the thimble preferably comprises a second, supplementary flange which complements the first flange of the interior surface of the proximal end of the thimble. Thus, the distal portion of the cannula is fastened inside the thimble.

The central portion of the cannula is preferably cylindrical and has a length suitable to introduce the thimble into the uterine cervix for sample collection.

The proximal portion of the cannula comprises guides which guide the sliding movement of a sheath (which is disclosed further below in this document) on the cannula. Moreover, it comprises a coupling means to couple the cannula to the sheath, which preferably consists of a fastening lip suitable to be inserted into complementary openings in the sheath.

3) A hollow sheath, which comprises a cylindrical distal portion the exterior surface whereof is smooth, and a proximal portion, the cannula and the thimble being lodged inside the sheath in a sliding manner, such that, in a first, retracted position, the thimble is completely inside the sheath and, in a second, extended position, the thimble protrudes through the distal end of the sheath.

The sheath covers the cannula and the thimble coupled thereto. It has a distal portion and a proximal portion:

The distal portion of the sheath is designed to be easily introduced through the patient's vagina until it reaches the uterine cervix and, for this reason, has a cylindrical shape with a smooth surface and an anatomically-shaped, semi-closed distal end, such that a slight pressure exercised by the thimble from the interior of the sheath opens the semi-closed distal end. Preferably, the semi-closed distal end has the shape of a set of petals.

The proximal portion of the sheath rotates with respect to the distal portion, and has a shape suitable for the patient to hold it with one hand. Preferably, it comprises supplementary coupling means which allow for coupling between the sheath and the cannula in two different positions, a first, retracted position and a second, extended position. Preferably, the supplementary coupling means are two openings located on the ends of the proximal portion of the sheath, whereto the cannula's coupling lip is coupled.

Thus, when the cannula is coupled to the sheath in the second, extended position, it is possible to rotate the proximal portion of the sheath; since the sheath is coupled to the cannula, and the latter is coupled to the thimble, which protrudes through the distal end of the sheath, this in turn causes rotation of the thimble; the latter then rubs against the uterine cervix walls, leading to the detachment of cells.

As is evident, all the parts that make up the cervical smear sampling device are made of biocompatible materials, preferably biocompatible plastic materials, and, more preferably, polyethylene.

Thus, the operation of the sampling device disclosed above comprises the following steps:
a) Introducing the cervical smear sampling device in a first, retracted position, wherein the thimble is completely inside the sheath, through the patient's vagina to the uterine cervix.
b) Extracting the sheath towards the exterior of the patient's body until the device is in a second, extended position, wherein the thimble protrudes through the distal end of the sheath.
c) Rotating the proximal portion of the sheath, such that the thimble rubs against the uterine cervix walls and some cells detach, sticking to the exterior surface of the thimble; and
d) Removing the smear sampling device from the vagina.

### DESCRIPTION OF THE DRAWINGS

In order to complement the description being made and in order to contribute to a better understanding of the characteristics of the invention, in accordance with a preferred embodiment example thereof, we attach, as an integral part of said description, a set of drawings which show the following for illustrative purposes, but not limited thereto:
Figures 1 and 2: Show two views of the thimble in accordance with this invention.
Figures 3 and 4: Show two views of the cannula in accordance with this invention.
Figures 5 and 6: Show two views of the sheath in accordance with this invention.
Figure 7: Shows a section wherein we can observe the fastening means between the thimble and the cannula's supplementary fastening means.
Figures 8 and 9: Show two perspective views of the coupling means between the sheath and the cannula.
Figures 10 and 11: Show the complete device, in the second position and in the first position, respectively.

### PREFERRED EMBODIMENT OF THE INVENTION

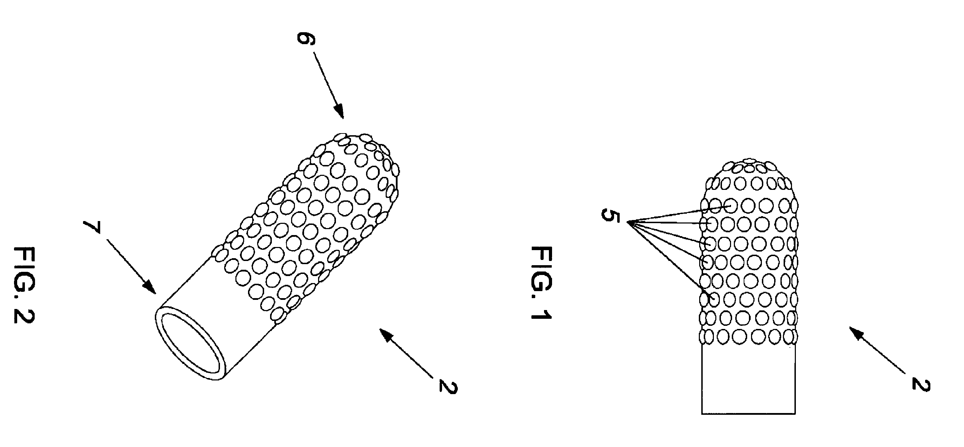

Below we disclose a particular example of a cervical smear sampling device, which is shown in Figures 1-11.

Figures 1 and 2 show a thimble (2) the exterior surface whereof is covered by a plurality of sample collection means (5), which in this example are semi-spherical protuberances, and the distal end (6) whereof is closed by an also semi-spherical surface. The material whereof the thimble (2) is made is a type of biocompatible polyethylene called Thermolast K HTF 8534/65, manufactured by Kraiburg TPE GMBH, and its dimensions are 40 mm in length by 15 mm in diameter.

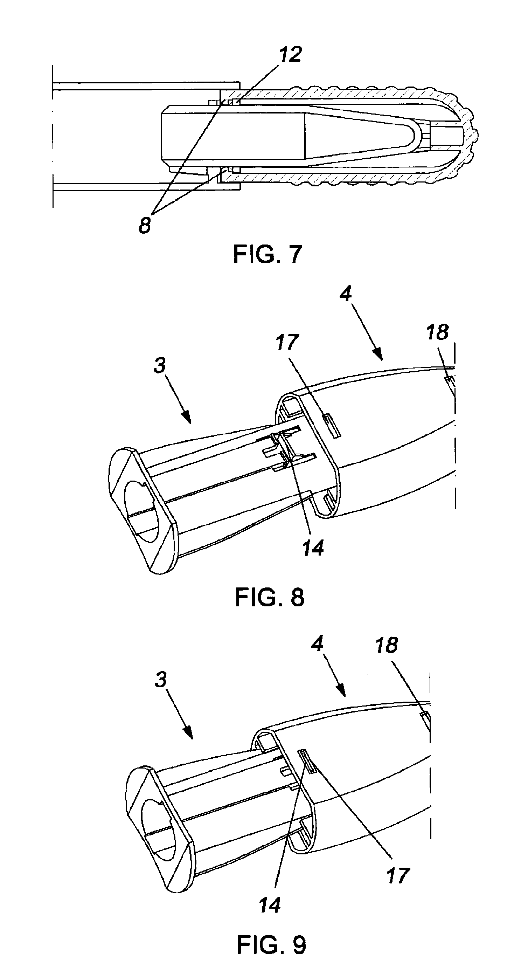

As has already been explained, the thimble (2) is the part of the device (1) which physically performs the sampling of uterine cervix cells when the sampling means (5) rub against the uterine cervix walls. For this reason, the material whereof it is made is soft to the touch and has a semi-spherical tip, and the exterior surfaces are soft in order to minimise discomfort for the patient. The interior surface of the thimble (2), in its proximal end (7), has a fastening means (8) to the cannula (3), which in this example is the flange shown in Figure 7.

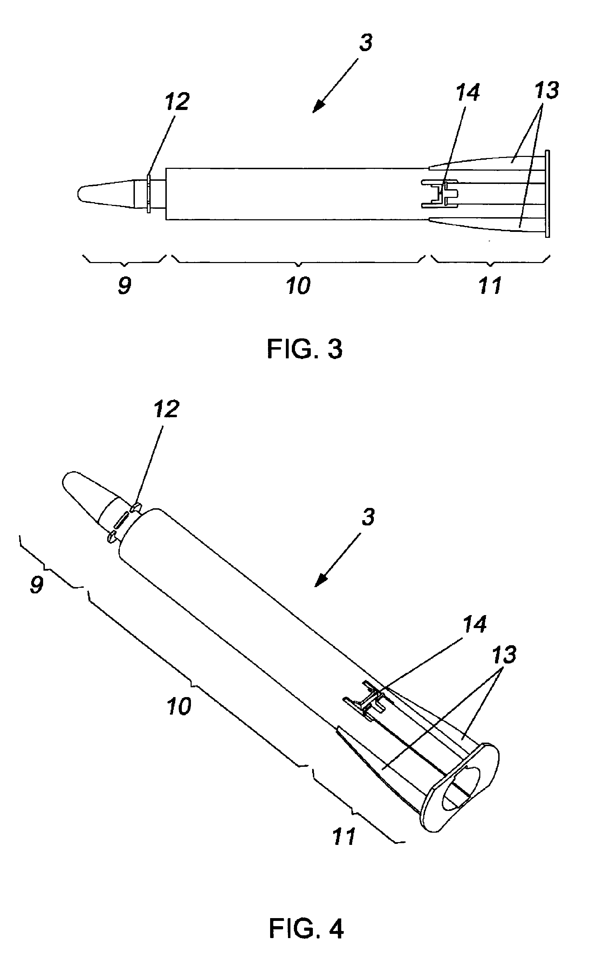

Figures 3 and 4 show a cannula (3) example in accordance with the invention. One may observe the three portions described above in this document. The shape of the distal portion (9), which is thinner and has an approximately conical end, is complementary to the interior shape of the thimble (2), with a supplementary fastening means (12), which in this example is a supplementary flange that couples to the flange of the thimble (2), as shown in Figure 7. The central portion (10) of the cannula (3) is a completely smooth cylinder. In the third place, the proximal portion (11) of the cannula (3) has a pair of guides (13) designed to guide sliding of the sheath (4) from the first, retracted position to the second, extended position, and vice-versa. Moreover, a coupling means (14), in this case a lip, allows the fastening of the cannula (3) to the sheath (4) such that undesirable relative movements are prevented upon introducing or removing the device (1) through the vagina.

The cannula (3) is made of biocompatible polypropylene, and the dimensions are 153 mm in total length by 17.4 mm in diameter in the central portion (10), and 30 mm in diameter in the proximal portion (11).

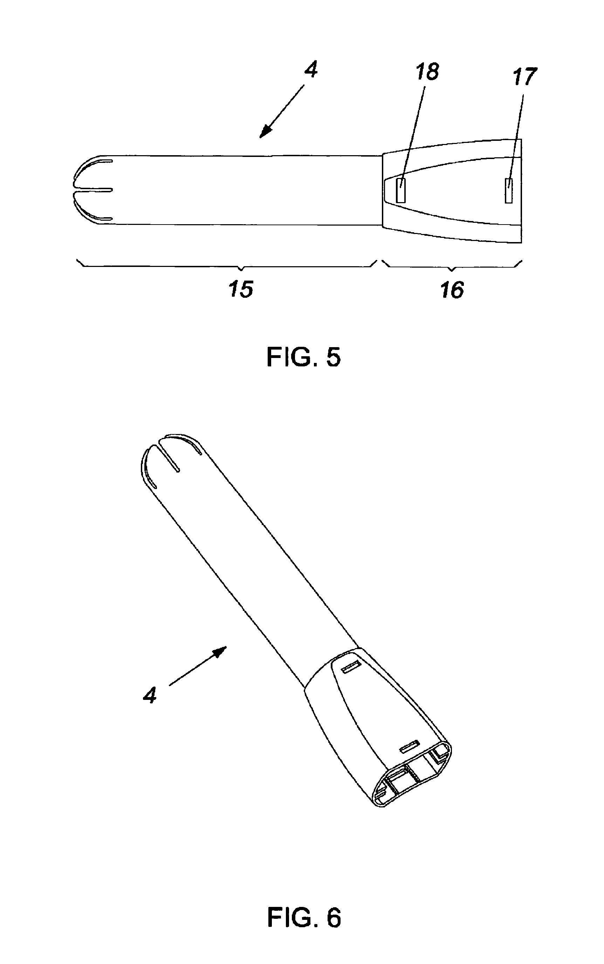

Figures 5 and 6 show a sheath (4) in accordance with the invention. One may observe the distal portion (15), which has a smooth cylindrical shape, and the proximal portion (16), which has a shape suitable to be held with the hands and two supplementary coupling means (17, 18), in this example two openings, whereon the lip that makes up the coupling means (14) of the cannula (3) may be fastened. Figures 8 and 9 show, in greater detail, the first opening (17) and the second opening (18), which correspond, when the coupling means (14) of the cannula (3) is introduced therein, to the first, retracted position and the second, extended position of the device (1) of the invention, respectively.

The total dimensions of the sheath (4) in this example are 130 mm in length by 20 mm in diameter in the distal portion (15), and 30 mm in diameter in the widest part of the proximal portion (16).

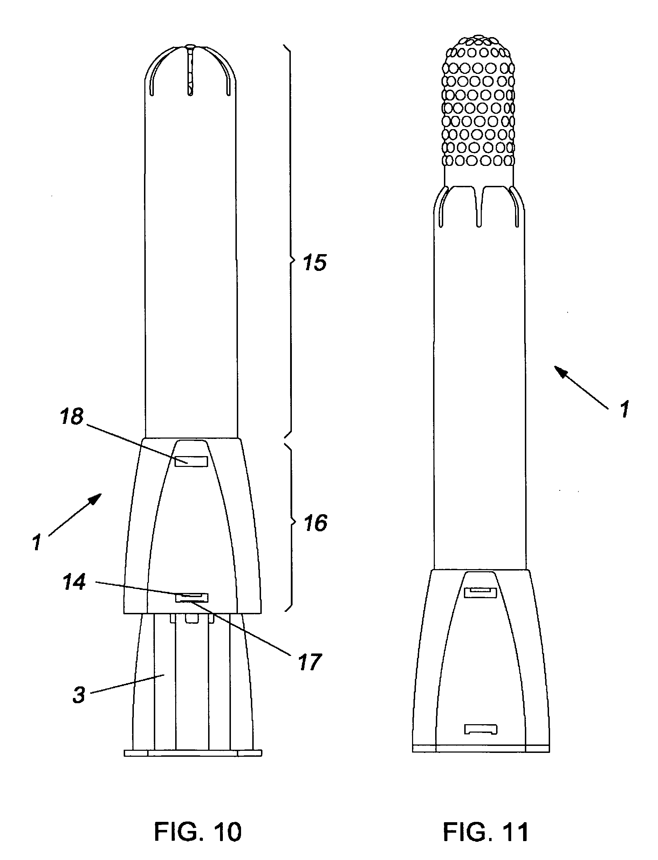

Thus, in order to use the device (1) of the invention, the patient holds it in the first, retracted position shown in Fig. 11 and carefully introduces the distal portion (15) of the sheath through her vagina until it reaches the uterine cervix, the proximal portion (16) of the sheath (4) and part of the cannula (3) remaining outside. Subsequently, by pressing the lip through the first opening (17), in order to release the coupling between the sheath (4) and the cannula (3), the patient softly pushes the cannula (3) whilst holding the proximal portion (16) of the sheath (4), such that the thimble (2) that is coupled to the cannula (3) slowly exits through the semi-closed distal end of the sheath (4), until the lip of the cannula (3) is introduced into the second opening (18) of the sheath (4), the device (1) remaining fastened in the second, extended position. The semi-closed distal end of the sheath (4), in this example, has an approximately semi-spherical anatomical shape formed by several sections that open up in a manner similar to flower petals when the thimble (2) slightly presses from the interior. The patient then rotates the proximal portion (16) of the sheath (4), which, since it is fastened to the cannula (3) and the latter is fastened to the thimble (2), causes a rotating movement in the thimble (2), which rubs against the uterine cervix walls.

One advantage of this device (1) is that, when the thimble (2) rotates, it is not necessary for the entire length of the sheath (4) to rotate, which in some cases could cause discomfort or even damage to the lips of the vagina or in the uterine cervix walls. By rotating the proximal portion (16) of the sheath (4), which is outside the vagina, the thimble (2), which is in the uterine cervix, is made to rotate, the distal portion (15) of the sheath remaining motionless.

Rubbing of the sampling means (5) against the uterine cervix walls causes cells to detach, which stick to the thimble (2). After rotating the proximal portion (16) of the sheath (4) for a few seconds, the patient removes the cervical smear sampling device (1).

## Claims

1. Cervical smear sampling device (1) to be used by patients themselves, which comprises:
a hollow, cylindrical thimble (2) the exterior surface whereof comprises sample collection means (5), and the distal end (6) whereof is closed by an anatomical surface;
an elongated cannula (3), formed by a distal portion (9), a central portion (10) and a proximal portion (11), the distal portion (9) whereof is coupled to the interior of the thimble (2); and
a hollow sheath (4), which comprises a cylindrical distal portion (15) the exterior surface whereof is smooth, and a proximal portion (16), the cannula (3) and the thimble (2) coupled thereto being lodged inside the sheath (4) in a sliding manner, such that, in a first, retracted position, the thimble (2) is completely inside the sheath (4) and, in a second, extended position, the thimble (2) protrudes through the distal end of the sheath (4),
**characterised in that** the proximal portion (11) of the cannula (3) comprises a coupling means (14) which may be coupled to supplementary coupling means (17, 18) of the proximal portion (16) of the sheath (4).

2. Sampling device (1) in accordance with claim 1, **characterised in that** the coupling means (14) is a lip that is fastened to the cannula (3).

3. Sampling device (1) in accordance with any of claims 1 or 2, **characterised in that** the supplementary coupling means (17, 18) of the sheath (4) are two openings located on the ends of the proximal portion (16) of the sheath (4).

4. Sampling device (1) in accordance with any of the previous claims, **characterised in that** the thimble (2) comprises a fastening means (8) that may be coupled to a supplementary fastening means (12) of the distal portion (9) of the sheath (4).

5. Sampling device (1) in accordance with claim 4, **characterised in that** the fastening means (8) of the thimble (2) is a flange that is located in the interior surface of its proximal end (7).

6. Sampling device (1) in accordance with claim 4, **characterised in that** the supplementary fastening means (12) of the cannula (3) is a flange that is located on its distal portion (9).

7. Sampling device (1) in accordance with any of the previous claims, **characterised in that** the anatomical surface of the thimble (2) is semi-spherical.

8. Sampling device (1) in accordance with any of the previous claims, **characterised in that** the sample collection means (5) have a semi-spherical shape.

9. Sampling device (1) in accordance with any of the previous claims, **characterised in that** the tip of the distal portion (15) of the sheath (4) has the shape of a set of petals.

10. Sampling device (1) in accordance with any of the previous claims, **characterised in that** the thimble (2), the cannula (3) and the sheath (4) are made of a biocompatible plastic material.

11. Sampling device (1) in accordance with claim 10, **characterised in that** the thimble (2), the cannula (3) and the sheath (4) are made of polyethylene.

## Patentansprüche

1. Gerät zur Entnahme von Portioabstrichen (1), das von den Patienten selbst angewendet werden kann, bestehend aus:
eine hohle zylinderförmige Hülse (2), wobei die Außenfläche Mittel zur Probenahme (5) umfasst, und deren distales Ende (6) mit einer anatomischen Fläche verschlossen ist;
eine längliche Kanüle (3), bestehend aus einem distalen Teil (9), einem mittleren Teil (10) und einem proximalen Teil (11), wobei das distale Teil (9) mit dem Innern der Hülse (2) verbunden ist; und
ein hohler Schaft (4), der ein zylinderförmiges distales Teil (15) umfasst, wobei dessen Außenfläche glatt ist, und ein proximales Teil (16), mit dem die Kanüle (3) und die Hülse (2) verbunden sind und das im Innern des Schafts (4) verschiebbar untergebracht ist, sodass sich die Hülse (2) in einer ersten zurückgezogenen Position vollständig im Innern des Schafts (4) befindet und in einer zweiten ausgefahrenen Position ragt die Hülse (2) aus dem distalen Ende des Schafts (4) heraus.
**dadurch gekennzeichnet, dass** das proximale Teil (11) der Kanüle (3) ein Verbindungselement (14) umfasst, das mit zusätzlichen Verbindungselementen (17, 18) des proximalen Teils (16) des Schafts (4) verbunden werden kann.

2. Probenahmegerät (1) nach Anspruch 1, **dadurch gekennzeichnet, dass** das Verbindungselement (14) eine Tülle ist, die an der Kanüle (3) befestigt ist.

3. Probenahmegerät (1) nach einem der Ansprüche 1 oder 2, **dadurch gekennzeichnet, dass** die zusätzlichen Verbindungselemente (17, 18) des Schafts (4) zwei Öffnungen darstellen, die sich an den Enden des proximalen Teils (16) des Schafts (4) befinden.

4. Probenahmegerät (1) nach einem der vorhergehenden Ansprüche, **dadurch gekennzeichnet, dass** die Hülse (2) ein Befestigungselement (8) umfasst, das mit einem zusätzlichen Befestigungselement (12) des distalen Teils (9) des Schafts (4) verbunden werden kann.

5. Probenahmegerät (1) nach Anspruch 4, **dadurch gekennzeichnet, dass** das Befestigungselement (8) der Hülse (2) eine Flansch ist, die sich an der Innenfläche seines proximalen Endes (7) befindet.

6. Probenahmegerät (1) nach Anspruch 4, **dadurch gekennzeichnet, dass** das zusätzliche Befestigungselement (12) der Kanüle (3) eine Flansch ist, die sich an seinem proximalen Ende (9) befindet.

7. Probenahmegerät (1) nach einem der vorhergehenden Ansprüche, **dadurch gekennzeichnet, dass** die anatomische Fläche der Hülse (2) halbkugelförmig ist.

8. Probenahmegerät (1) nach einem der vorhergehenden Ansprüche, **dadurch gekennzeichnet, dass** das Probenahmeelement (5) eine halbkugelförmige Gestalt hat.

9. Probenahmegerät (1) nach einem der vorhergehenden Ansprüche, **dadurch gekennzeichnet, dass** die Spitzes des distalen Teils (15) des Schafts (4) die Form einer Blumenblattanordnung hat.

10. Probenahmegerät (1) nach einem der vorhergehenden Ansprüche, **dadurch gekennzeichnet, dass** die Hülse (2), die Kanüle (3) und der Schaft (4) aus einem biokompatiblen Kunststoffmaterial hergestellt sind.

11. Probenahmegerät (1) nach Anspruch 10, **dadurch gekennzeichnet, dass** die Hülse (2), die Kanüle (3) und der Schaft (4) aus Polyethylen hergestellt sind.

## Revendications

1. Dispositif de prélèvement (1) de glaire cervicale à utiliser par les patientes elles-mêmes, qui comprend :
un dé creux cylindrique (2) dont la surface extérieure comprend des moyens de prélèvement d'échantillons (5), et dont l'extrémité distale (6) est fermée par une surface anatomique ;
une canule allongée (3), formée par une portion distale (9), une portion centrale (10) et une portion proximale (11), dont la portion distale (9) est couplée à l'intérieur du dé (2) ; et
une gaine creuse (4), qui comprend une portion distale cylindrique (15) dont la surface extérieure est lisse, et une portion proximale (16), la canule (3) et le dé (2) couplés à celle-ci étant logés à l'intérieur de la gaine (4) de manière coulissante, de telle façon que, dans une première, position rétractée, le dé (2) est complètement à l'intérieur de la gaine (4) et, dans une seconde, position déployée, le dé (2) dépasse de l'extrémité distale de la gaine (4),
**caractérisé en ce que** la portion proximale (11) de la canule (3) comprend un moyen de couplage (14) qui peut être couplé à des moyens de couplage supplémentaires (17, 18) de la portion proximale (16) de la gaine (4).

2. Dispositif de prélèvement (1) conformément à la revendication 1, **caractérisé en ce que** le moyen de couplage (14) est une lèvre qui est fixée à la canule (3).

3. Dispositif de prélèvement (1) conformément à n'importe laquelle des revendications 1 ou 2, **caractérisé en ce que** les moyens de couplage supplémentaires (17, 18) de la gaine (4) sont deux ouvertures situées sur les extrémités de la portion proximale (16) de la gaine (4).

4. Dispositif de prélèvement (1) conformément à n'importe laquelle des revendications précédentes, **caractérisé en ce que** le dé (2) comprend un moyen de fixation (8) qui peut être couplé à un moyen de fixation supplémentaire (12) de la potion distale (9) de la gaine (4).

5. Dispositif de prélèvement (1) conformément à la revendication 4, **caractérisé en ce que** le moyen de fixation (8) du dé (2) est une collerette qui est située sur la surface interne de son extrémité proximale (7).

6. Dispositif de prélèvement (1) conformément à la revendication 4, **caractérisé en ce que** le moyen de fixation supplémentaire (12) de la canule (3) est une collerette qui est située sur sa portion distale (9).

7. Dispositif de prélèvement (1) conformément à n'importe laquelle des revendications précédentes, **caractérisé en ce que** la surface anatomique du dé (2) est semi-sphérique.

8. Dispositif de prélèvement (1) conformément à n'importe laquelle des revendications précédentes, **caractérisé en ce que** le moyen de prélèvement d'échantillons (5) a une forme semi-sphérique.

9. Dispositif de prélèvement (1) conformément à n'importe laquelle des revendications précédentes, **caractérisé en ce que** le bout de la portion distale (15) de la gaine (4) a la forme d'un ensemble de pétales.

10. Dispositif de prélèvement (1) conformément à n'importe laquelle des revendications précédentes, **caractérisé en ce que** le dé (2), la canule (3) et la gaine (4) sont fabriqués dans un matériau plastique biocompatible.

11. Dispositif de prélèvement (1) conformément à la revendication 10, **caractérisé en ce que** le dé (2), la canule (3) et la gaine (4) sont fabriqués en polyéthylène.
